# EUROPÄISCHE PATENTSCHRIFT

(11) **EP 1 061 978 B1**
(45) Veröffentlichungstag und Bekanntmachung des Hinweises auf die Patenterteilung: **12.05.2004**
(21) Anmeldenummer: 99963271.4
(22) Anmeldetag: 27.11.1999
(51) Int. Cl.: A61M 5/168

(54) **INJEKTOR ZUR APPLIZIERUNG VON FLÜSSIGKEITEN, INSBESONDERE VON KONTRASTMITTELN FÜR DIE RÖNTGEN- UND KERNSPINTOMOGRAPHIE**
INJECTOR FOR APPLYING FLUIDS, ESPECIALLY CONTRAST AGENTS IN X-RAY AND NUCLEAR SPIN TOMOGRAPHY
INJECTEUR POUR L'APPLICATION DE LIQUIDES, EN PARTICULIER D'AGENTS DE CONTRASTE EN TOMOGRAPHIE AUX RAYONS X OU A SPIN NUCLEAIRE

(30) Priorität: 13.01.1999 DE 19900936
(43) Veröffentlichungstag der Anmeldung: 27.12.2000
(73) Patentinhaber: Ulrich GmbH & Co. KG, 89081 Ulm (DE)
(72) Erfinder: FUTTERKNECHT, Hans-Dieter, D-89075 Ulm (DE)
(74) Vertreter: Hentrich, Swen Dipl.-Phys. Dr.
(86) Internationale Anmeldenummer: PCT/DE1999/003795
(87) Internationale Veröffentlichungsnummer: WO 2000/041751

(56) Entgegenhaltungen:
- WO-A-96/40330
- WO-A-97/45150
- FR-A- 2 163 893
- US-A- 3 677 248
- US-A- 4 710 166

## Beschreibung

Die Erfindung betrifft einen Injektor zur Applizierung von Flüssigkeiten, insbesondere von Kontrastmitteln für die Röntgen- und Kernspintomographie, mit einem aus Verbindungsschläuchen und einem Pumpenschlauch bestehenden Schlauchsystem und mit einer Mehrzahl von Vorratsgefäßen, die über je einen der Verbindungsschläuche sowie ein Verzweigungsstück an dem zu einer Kanüle führenden Pumpenschlauch angeschlossen sind.

Bei derartigen aus der Praxis bekannten Injektoren sind mehrere Vorratsgefäße an einem Rahmengestell des Injektors gehalten, die insgesamt ein Volumen zur Verfügung stellen, das ausreicht, um an einem Arbeitstag oder innerhalb einer Arbeitsschicht die notwendigen Untersuchungen an einer Vielzahl von Patienten durchführen zu können, ohne daß oftmals Unterbrechungspausen eintreten, in denen die Flüssigkeit ersetzt bzw. das Vorratsgefäß ausgewechselt werden muß. Dieser Wechselvorgang selber ist relativ aufwendig, da das zu ersetzende Vorratsgefäß von dem Verbindungsschlauch gelöst werden muß und dabei unvermeidlich Luft in den Verbindungsschlauch eindringt und dort eine Luftblase bildet. Das gleiche Problem tritt auch auf, wenn während der Untersuchungen ein Vorratsgefäß leerläuft und so Luft in den Verbindungsschlauch eindringt, die nicht bis zu dem Patienten vordringen und durch eine Vene eingespritzt werden darf, weil dies mit dem Risiko von Embolien verbunden ist.

US-A-4 710 166 zeigt ein Injektorsystem wie im Oberbegriff vom Anspruch 1. Ein Gasblasen detektor kommt jedoch nur in einem der Verbindungsschläuche vor und eine stromauf Pumpenrichtung kommt gemäß Spatte 9 Zeile 22 im US-Dokument nie vor.

Der Erfindung liegt daher die Aufgabe zugrunde, einen Injektor der eingangs genannten Art so auszubilden, daß das Eindringen von Blasen in den Pumpenschlauch in jedwedem Betriebszustand, insbesondere auch beim Auswechseln der Vorratsgefäße sicher vermieden wird.

Diese Aufgabe wird nach der Erfindung bei einem Injektor der eingangs genannten Art dadurch gelöst, daß jedem Verbindungsschlauch ein Gasblasendetektor sowie ein Ventil zugeordnet sind und daß dem Pumpenschlauch ein den Flüssigkeitsstrom vom Vorratsgefäß zur Kanüle umkehrendes Pumporgan zugeordnet ist.

Dieser Injektor bietet den Vorteil, daß das Eindringen einer Luftblase in den Verbindungsschlauch, beispielsweise wenn das Vorratsgefäß leergelaufen ist, detektiert wird, woraufhin mittels des zugeordneten Ventils der die Luftblase enthaltende Verbindungsschlauch abgesperrt und ein anderer Verbindungsschlauch mittels dessen Ventil freigeschaltet werden kann, so daß die Untersuchung des Patienten ohne Zeitverzögerung fortsetzbar ist. Außerdem ist es mit dem erfindungsgemäßen Injektor möglich, nicht nur die Präsenz der Luftblase im Verbindungsschlauch zu detektieren, vielmehr bietet das Pumporgan nach dem Ersatz des leergelaufenen Vorratsgefäßes durch ein gefülltes die Möglichkeit, das zuvor geschlossene Ventil wieder zu öffnen und auf die im Pumpenschlauch und dem Verbindungsschlauch befindliche Flüssigkeit einen Druckstoß auszuüben, durch den die Flüssigkeit stromauf verschoben wird, wodurch die Luftblase aus dem Verbindungsstück in das neue Vorratsgefäß zurückgepreßt wird und im Vorratsgefäß nach oben aufsteigen kann, so daß im Ergebnis der Verbindungsschlauch wieder durchgehend mit der Flüssigkeit befüllt ist und das gewechselte Vorratsgefäß eingesetzt werden kann.

Gemäß Ausführungsbeispiele ist weiterhin vorgesehen, daß dem Pumpenschlauch zur aktiven Förderung der Flüssigkeit vom Vorratsgefäß zur Kanüle eine Rollenpumpe zugeordnet ist. Dies bietet den Vorteil, daß nicht nur die Schwerkraft ausgenutzt werden muß, um gegen den Venendruck die Flüssigkeit in den Patienten zu injizieren, wobei weiterhin durch Veränderung der Drehzahl der Rollenpumpe die Flüssigkeitsmenge variiert werden kann, die pro vorgegebener Zeiteinheit injiziert wird. Die Verwendung einer Rollenpumpe bietet dabei weiterhin den besonderen Vorteil, daß kein unmittelbarer Kontakt zwischen der im Inneren des Pumpenschlauches angeordneten Flüssigkeit und der Rollenpumpe erfolgt, die Sterilität der Flüssigkeit also jederzeit gewährleistet ist und auch keine Gefahr der Kontamination der Flüssigkeit besteht.

Als zweckmäßig hat es sich gezeigt, wenn der Gasblasendetektor durch einen Ultraschallsender und -empfänger gebildet ist, durch dessen Signale das zugeordnete Ventil schaltbar ist. Ein derartig geschalteter Gasblasendetektor hat für den gewünschten Einsatzzweck eine ausreichende Genauigkeit und Auflösungsvermögen und liefert elektrische Signale, die mit den üblichen Mitteln zur Signalverarbeitung genutzt werden können, um das zugeordnete Ventil zu schalten.

Zweckmäßigerweise sind die Ventile durch Schlauchklemmen gebildet, die den Querschnitt der Verbindungsschläuche so verengen, daß dort keine Flüssigkeit mehr hindurchtreten kann. Die außen an den Verbindungsschläuchen angreifenden Schlauchklemmen bieten dabei die zuvor schon bei der Rollenpumpe genannten Vorteile, daß die Sterilität der Flüssigkeit jederzeit gewährleistet ist, weil in den Flüssigkeitsstrom selber keine Bauteile eingreifen müssen, um diesen zu unterbrechen.

Gemäß einem Ausführungsbeispiel ist vorgesehen, daß die Schlauchklemmen einen den Verbindungsschlauch gegen ein Widerlager pressenden und dadurch den Querschnitt verengenden verstellbaren Stempel aufweisen. Die Verwendung eines Stempels zur Sperrung des Flüssigkeitstromes ist günstiger als die einer prinzipiell auch verwendbaren Schlauchschelle, die nicht so schnell verstellt werden kann und einem größeren Verschleiß unterliegt.

Zur besseren Überwachung des Injektionsvorganges weisen die Schlauchklemmen einen die Stellung des Stempels erfassenden Schalter auf.

Nach einer ganz besonders bevorzugten Ausführungsform ist weiterhin vorgesehen, daß das Pumporgan ein am Pumpenschlauch anliegendes Stellglied aufweist, durch das für den Pumpvorgang der Pumpenschlauch verformbar und im Inneren des Schlauches die stromauf vom Stellglied stehende Flüssigkeit stromauf preßbar ist. Bei dieser Ausgestaltung des Pumporganes ist zunächst feststellbar, daß wiederum jeder unmittelbare Kontakt mit der Flüssigkeit vermieden ist, weil das Pumporgan lediglich am Außenumfang des Pumpenschlauches angreift. Vorteilhaft an dieser Ausgestaltung des Pumporganes ist auch, daß es mechanisch einfach aufgebaut und so preisgünstig herzustellen ist und keine Störanfälligkeit im Betrieb zeigt. Aufgrund der Konstruktion des Pumporganes ist auch sichergestellt, daß der Flüssigkeitsstrom nur kurzfristig im Pumpenschlauch umgekehrbar ist, im Schlauchsystem also kein Unterdruck erzeugt werden kann, der einen langanhaltenden Sog auf die Vene des Patienten ausübt. Der nur kurzfristig durch das Pumporgan erzeugte Druckstoß ist aber völlig ausreichend, um die stromauf liegende Flüssigkeitssäule geringfügig soweit zu verschieben, daß die im Verbindungsschlauch eingetretene Luftblase wieder aus diesem Verbindungsschlauch entfernt wird. Selbstverständlich ist es unter Inkaufnahme des höheren apparativen Aufwandes auch möglich, das Pumporgan jedem Verbindungsschlauch statt dem Pumpenschlauch zuzuordnen.

Einen besonders einfachen Aufbau hat das Pumporgan, wenn das Stellglied durch eine einseitig befestigte W-förmige Feder gebildet ist, deren am freien Ende angeordneter Schenkel aus einer geneigt zum Pumpenschlauch orientierten Ruhelage in eine parallel zum Pumpenschlauch verlaufende Lage verstellbar und gegen den Pumpenschlauch preßbar ist. Bei dieser Gestaltung des Stellgliedes wird bei dessen Betätigung zunächst der Pumpenschlauch punktuell verformt und der Flüssigkeitsstrom stromab gestoppt, wobei bei der anschließenden Anlage des freien Schenkel die stromauf vor der gesperrten Stelle liegende Flüssigkeit Richtung Vorratsgefäß gedrückt wird.

Prinzipiell ist es möglich, daß durch das Stellglied der frei hängende Pumpenschlauch so weit verstellt wird, bis eine weitere Biegung nicht möglich ist und die Abklemmung einsetzt. Günstiger ist es aber, wenn auf der dem Stellglied gegenüberliegenden Seite des Pumpenschlauches als Gegenlager eine Platte angeordnet ist, da so der Verschleiß von dem Pumpenschlauch reduziert wird.

Es hat sich als zweckmäßig gezeigt, wenn ein zweimarmiger Hebel, dessen einer Arm in der Ruhelage dem Schenkel am freien Ende der Feder anliegt und dessen anderer Arm mit einem Stellmotor gekoppelt ist, zur Verstellung des Stellgliedes vorgesehen ist.

Als günstig hat es sich weiterhin gezeigt, wenn im Schlauchsystem eine Druckkammer integriert ist, die durch mindestens eine in der Schlauchwandung ausgebildete Öffnung mit dem Inneren des Pumpenschlauches verbunden und von der Flüssigkeit befüllbar ist, und wenn die Druckkammer ein unter der Wirkung des Flüssigkeitsdruckes verstellbares Bauteil aufweist, das auf einen Drucksensor einwirkt. Durch die Überwachung des Druckes im Schlauchsystem ist es möglich, genau zu bestimmen, wieviel Flüssigkeit dem Patienten tatsächlich injiziert wird. Weiterhin bildet die Druckmessung eine weitere Sicherheitseinrichtung, da Störungen im Betrieb sich in der Regel stets auf den im Schlauchsystem herrschenden Druck auswirken, beispielsweise wenn durch eine Fehlfunktion der Ventile sämtliche Verbindungsschläuche geschlossen werden und dadurch der Druck im Schlauchsystem abfällt, oder wenn durch Verwendung einer unzulässigen Kanüle mit zu geringem Querschnitt der Gegendruck zu groß wird und daher auch der Druck im Schlauchsystem. Auch Undichtigkeiten des Schlauchsystemes sind durch einen Druckabfall feststellbar.

Da der Pumpenschlauch zur Verwendung in Kombination mit einer Rollenpumpe eine ausreichende Elastizität aufweisen muß, um den Flüssigkeitsstrom vom Vorratsgefäß zur Kanüle zu fördern, diese Elastizität bei der Druckmessung sich aber nachteilig auswirkt, ist es vorgesehen, daß zwischen zwei Abschnitten des Pumpenschlauches ein starres, die Öffnung aufweisendes Rohr eingesetzt ist, an dem die Druckkammer befestigt ist.

Um einen unmittelbaren Kontakt zwischen der Flüssigkeit und dem Drucksensor zu verhindern, ist es in einfacher Weise möglich, daß das verstellbare Bauteil durch eine flüssigkeitsdichte, elastische Membran gebildet ist.

Alternativ besteht die Möglichkeit, daß das verstellbare Bauteil als gegenüber der Druckkammerwandung abdichtender Kolben ausgebildet ist, der unmittelbar auf den Drucksensor einwirkt.

Da durch das Pumporgan die im Schlauchsystem befindliche Flüssigkeit stromauf verschoben wird, wird am stromab gelegenen Ende des Schlauchsystemes ein Sog erzeugt. Um mit Sicherheit zu verhindern, daß Blut des Patienten in das Schlauchsystem eindringen kann, das gesamte Schlauchsystem also bei jedem Wechsel des Patienten gleichfalls gewechselt werden muß, um die hygienische Unbedenklichkeit zu gewährleisten, ist vorgesehen, daß der Pumpenschlauch zweiteilig ausgebildet ist, und daß der das freie Ende bildende Teil mindestens ein Rückschlagventil aufweist. Bei dieser Ausführungsform ist sichergestellt, daß nur der Patientenschlauch selber gewechselt werden muß, das übrige Schlauchsystem aber frei von einer Kontamination bleibt, wie sich bei einer hygienischen Überprüfung gezeigt hat.

Im folgenden soll die Erfindung an in der Zeichnung dargestellten Ausführungsbeispielen näher erläutert werden; es zeigen:

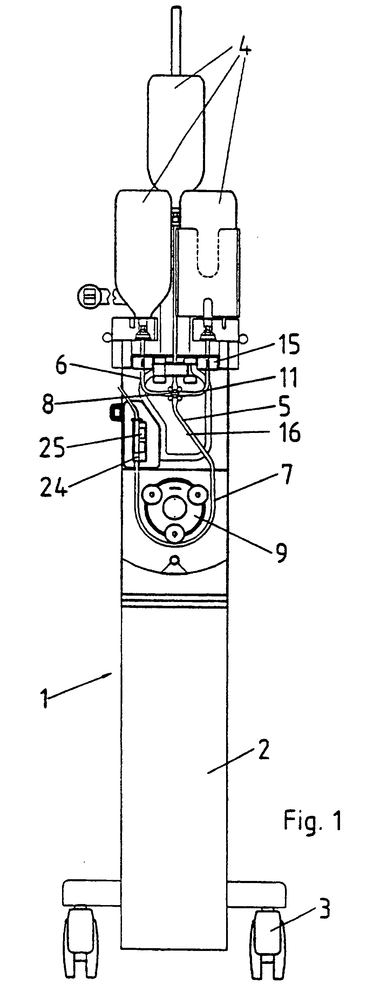

- Fig. 1: eine perspektivische Darstellung des erfindungsgemäßen Injektors, mit den Vorratsgefäßen, dem Schlauchsystem, sowie der Rollenpumpe,

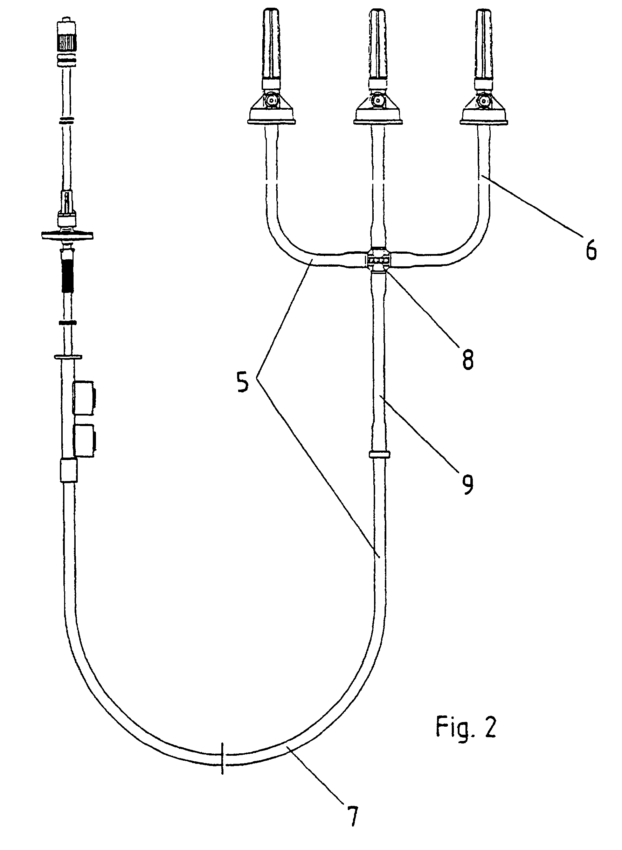

- Fig. 2: eine isolierte Darstellung des Schlauchsystemes,
- Fig. 3: eines der den Verbindungsschläuchen des Schlauchsystemes zugeordneten Ventile in der geöffneten Stellung,
- Fig. 4: das Ventil aus Fig. 3 in der geschlossenen Stellung,
- Fig. 5: eine Seitenansicht des W-förmigen Stellgliedes des Pumporgans in der Ruhelage,
- Fig. 6: eine der Fig. 5 entsprechende Darstellung mit dem den Pumpenschlauch sperrenden Stellglied des Pumporgans,

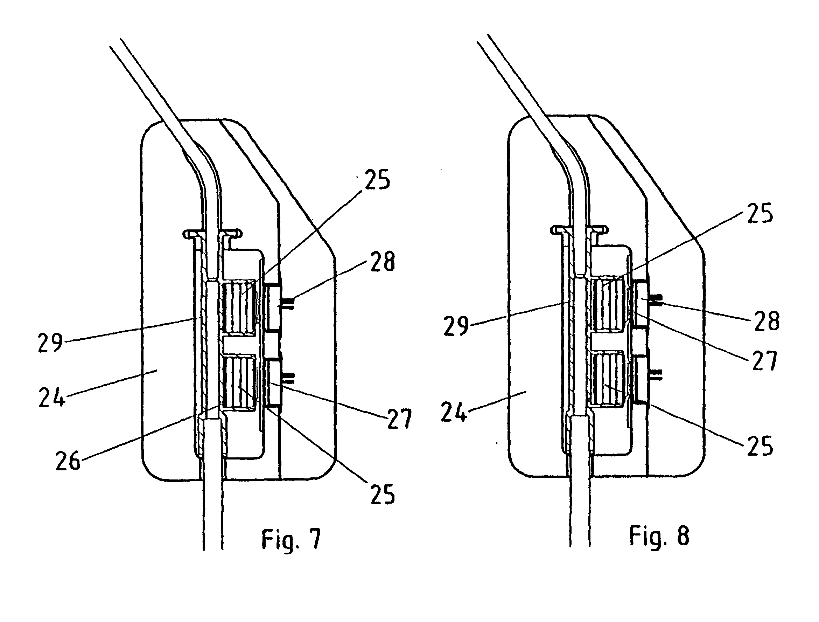

- Fig. 7: das dem Pumpenschlauch zugeordnete, aus einer Druckkammer und einem Drucksensor bestehende Druckmeßsystem in einer Seitenansicht, mit einer drucklosen Druckkammer, und
- Fig. 8: eine der Fig. 7 entsprechende Darstellung des Druckmeßsystems mit dem maximal verstellten Kolben.

Der in der Zeichnung dargestellte Injektor 1 dient insbesondere zur Applizierung von Kontrastmitteln für röntgen- oder kernspintomographische Untersuchungen an Patienten, denen über einen längeren Zeitraum kontinuierlich mit konstanter oder variierender Menge ein Kontrastmittel oder im Wechsel eine NaCl-Lösung zum Spülen zugeführt werden soll. Der Injektor 1 besitzt ein Rahmengestell 2, das in der in der Zeichnung dargestellten Ausführungsform in bekannter Weise auf Rollen 3 gelagert ist, aber auch an einem stativarm an der Wand oder Decke in der Nähe des Untersuchungsgerätes befestigt sein kann. An dem Rahmengestell 2 der in der Zeichnung dargestellten Ausführungsform sind drei Vorratsgefäße 4 gehalten, aus denen die Flüssigkeit über ein Schlauchsystem 5 dem Patienten zugeführt wird. Das Schlauchsystem 5 besteht aus an den Vorratsgefäßen 4 angeschlossenen Verbindungsschläuchen 6 sowie einem Pumpenschlauch 7, der über ein Verzweigungsstück 8 mit den Verbindungsschläuchen 6 gekoppelt ist.

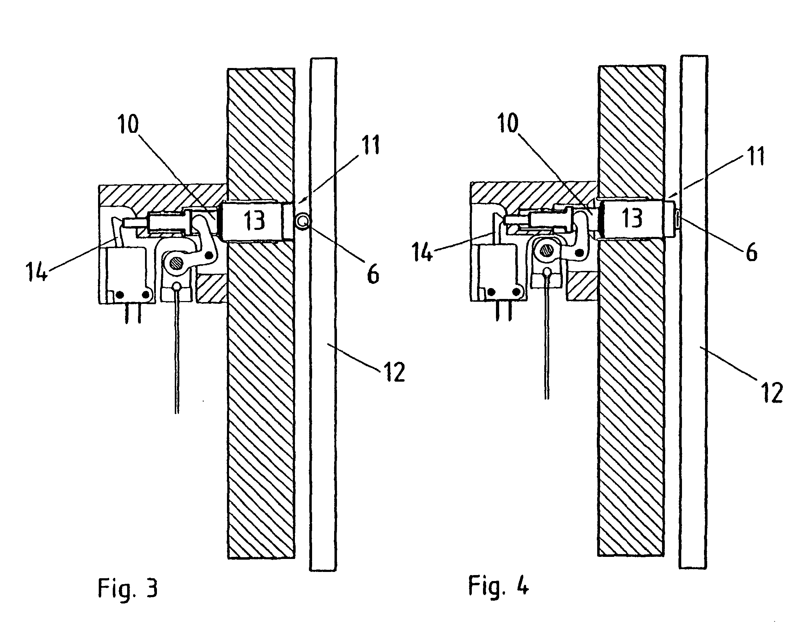

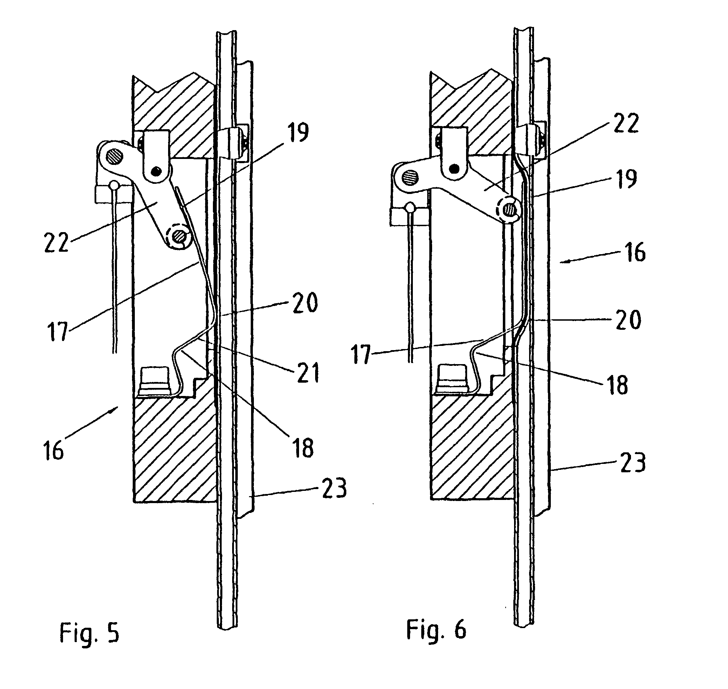

Über eine Rollenpumpe 9, die am Außenumfang des Pumpenschlauches 7 angreift und nicht in unmittelbarem Kontakt mit dem Kontrastmittel steht, wird dieses von den Vorratsgefäßen 4 zum Patienten gefördert. Damit nicht sämtliche Vorratsgefäße 4 gleichzeitig geleert werden, ist jedem Verbindungsschlauch 6 ein durch eine Schlauchklemme 10 gebildetes Ventil 11 zugeordnet, das eine den Verbindungsschlauch 6 gegen ein Widerlager 12 pressenden Stempel 13 aufweist, durch den der Verbindungsschlauch 6 abgesperrt werden kann. Der Stempel 13 wird motorisch aus der in Fig. 3 gezeigten Stellung mit geöffnetem Ventil 11 in die in Fig. 4 gezeigte geschlossene Stellung verstellt, wobei die Stellung des Stempels 13 durch einen Schalter 14 überwacht wird, der über Signalleitungen mit einer Auswerte- und Steuereinheit verbunden ist, die auch die Ventile 11, das Pumporgan 16 und die Rollenpumpe 9 schaltet. Mit diesen Ventilen 11 besteht die Möglichkeit, zunächst einen Verbindungsschlauch 6 freizugeben und aus einem Vorratsgefäß 4 Flüssigkeit zu entnehmen, während die anderen beiden gefüllt bleiben. Läuft nun das in Benutzung befindliche Vorratsgefäß 4 leer, tritt eine Luftblase in den Verbindungsschlauch 6 ein. Diese Luftblase wird durch einen Gasblasendetektor 15 detektiert, der durch einen Ultraschallsender und -empfänger gebildet ist, wobei die vom Empfänger erzeugten elektrischen Signale genutzt werden, das dem Verbindungsschlauch 6 zugeordnete Ventil 11 zu schalten, also das weitere Vordringen der Luftblase zu unterbinden. Zugleich besteht die Möglichkeit, das Ventil 11 des anderen, gefüllten Vorratsgefäßes 4 zu öffnen, und so einen kontinuierlichen Strom der Flüssigkeit zu gewährleisten. Das leere Vorratsgefäß 4 kann nun in einfacher Weise durch ein gefülltes ersetzt werden. Allerdings ist ein erneutes einfaches Umschalten zwischen den Vorratsgefäßen 4 nicht möglich, weil sich noch die Luftblase im Verbindungsschlauch 6 befindet und weiter transportiert würde. Um die Luftblase auch aus dem Schlauchsystem 5 zu entfernen, ist dem Pumpenschlauch 7 ein den Flüssigkeitsstrom vom Vorratsgefäß 4 zur Kanüle umkehrendes Pumporgan 16 zugeordnet, das ein am Pumpenschlauch 7 anliegendes Stellglied 17 aufweist, durch das für den Pumpvorgang der Pumpenschlauch 7 verformbar und im Inneren des Pumpenschlauches 7 die stromauf vom Stellglied stehende Flüssigkeit stromauf preßbar ist. Die im Pumpenschlauch 7 und in dem Verbindungsschlauch 6 stehende Flüssigkeitssäule wird also durch das Pumporgan 16 in Richtung des Vorratsgefäßes 4 verschoben, so daß die Luftblase aus dem Verbindungsschlauch 6 austritt und in das Vorratsgefäß 4 eintritt und dort an die Oberfläche aufsteigt, so daß einer erneuten Verwendung des Verbindungsschlauches 6 und des Vorratsgefäßes 4 nichts entgegensteht. Das Pumporgan 16 ist in den Fig. 5 und 6 detaillierter dargestellt. Wie daraus ersichtlich ist, ist das Stellglied 17 durch eine einseitig befestigte W-förmige Feder 18 gebildet, die an einem Ende fixiert ist. Der am freien Ende angeordnete Schenkel 19 ist aus einer geneigt zum Pumpenschlauch 7 orientierte Ruhelage, in der er nur mit dem Verbindungsbogen 20 zum benachbarten Schenkel 21 an dem Pumpenschlauch 7 anliegt, in eine parallel zum Pumpenschlauch 7 verlaufende Lage verstellbar, in der er durch einen zweiarmigen Hebel 22 den Pumpenschlauch 7 gegen eine als Widerlager wirkende Platte 23 preßt. Zu beachten ist dabei, daß bei der anfänglichen Verstellung der Feder 18 zunächst der Pumpenschlauch 7 abgeklemmt wird und bei der nachfolgenden Verstellung die vor der Klemmstelle stromauf liegende Flüssigkeit von dem freien Schenkel 19 der Feder 18 in Richtung Vorratsgefäß 4 verschoben wird. Mit dem zweiten Arm des zweiarmigen Hebel 22 ist ein Stellmotor verbunden.

Um die im Schlauchsystem 5 herrschenden Druckverhältnisse kontinuierlich überwachen zu können, ist im Schlauchsystem 5 ein Druckmeßsystem 24 mit einer Druckkammer 25 integriert, die durch eine in der Schlauchwandung ausgebildete Öffnung 26 mit dem Inneren des Pumpenschlauches 7 verbunden und von der Flüssigkeit durchströmbar ist, wobei die Druckkammer 25 ein unter der Wirkung des Flüssigkeitsdruckes verstellbares Bauteil 27 aufweist, das auf einen Drucksensor 28 einwirkt, der so die herrschenden Druckverhältnisse erfaßt. Dabei ist die Druckkammer 25 an einem starren, die Öffnung 26 aufweisenden Rohr 29 befestigt, das zwischen zwei Abschnitten des Pumpenschlauches 7 eingesetzt ist.

Das verstellbare Bauteil ist nach einer nicht in der Zeichnung dargestellten Ausführungsform durch eine flüssigkeitsdichte, elastische Membran gebildet, während bei der in der Zeichnung dargestellten Ausführungsform das verstellbare Bauteil 27 als gegenüber der Druckkammerwandung abdichtender Kolben ausgebildet ist, der unmittelbar auf den Drucksensor 28 einwirkt und den Vorteil eines größeren Verstellweges hat und so eine größere Meßgenauigkeit ermöglicht.

Das Schlauchsystem 5 ist weiterhin so gestaltet, daß der Pumpenschlauch 7 zweiteilig ausgebildet ist, wobei der das freie Ende bildende Teil ein Rückschlagventil aufweist, das das Eindringen von Blut in das Schlauchsystem 5 verhindert.

## Patentansprüche

1. Injektor zur Applizierung von Flüssigkeiten, insbesondere von Kontrastmitteln für die Röntgen- und Kernspintomographie, mit einem aus Verbindungsschläuchen (6) und einem Pumpenschlauch (7 bestehenden Schlauchsystem (5) und mit einer Mehrzahl von Vorratsgefäßen (4), die über je einen der Verbindungsschläuche (6) sowie ein Verzweigungsstück (8) an dem zu einer Kanüle führenden Pumpenschlauch (7) angeschlossen sind, **dadurch gekennzeichnet, daß** jedem Verbindungsschlauch (6) ein Gasblasendetektor (15) sowie ein Ventil (11) zugeordnet sind, und daß dem Pumpenschlauch (7) ein den Flüssigkeitsstrom vom Vorratsgefäß (4) zur Kanüle umkehrendes Pumporgan (16) zugeordnet ist.

2. Injektor nach Anspruch 1, **dadurch gekennzeichnet, daß** dem Pumpenschlauch (7) zur aktiven Förderung der Flüssigkeit vom Vorratsgefäß (4) zur Kanüle eine Rollenpumpe (9) zugeordnet ist.

3. Injektor nach Anspruch 1 oder 2, **dadurch gekennzeichnet, daß** der Gasblasendetektor (15) durch einen Ultraschallsender und -empfänger gebildet ist, durch dessen Signale das zugeordnete Ventil (11) schaltbar ist.

4. Injektor nach einem der Ansprüche 1 bis 3, **dadurch gekennzeichnet, daß** die Ventile (11) durch Schlauchklemmen (10) gebildet sind.

5. Injektor nach Anspruch 4, **dadurch gekennzeichnet, daß** die Schlauchklemmen (10) einen den Verbindungsschlauch (6) gegen ein Widerlager (12) pressenden und dadurch den Querschnitt verengenden verstellbaren Stempel (13) aufweist.

6. Injektor nach Anspruch 5, **dadurch gekennzeichnet, daß** die Schlauchklemme (10) einen die Stellung des Stempels (12) erfassender Schalter (14) aufweist.

7. Injektor nach einem der Ansprüche 1 bis 6, **dadurch gekennzeichnet, daß** das Pumporgan (16) ein am Pumpenschlauch (7) anliegendes Stellglied (17) aufweist durch das für den Pumpvorgang der Pumpenschlauch (7) auslenkbar und im Inneren des Pumpenschlauches (7) die stromauf vom Stellglied (17) stehende Flüssigkeit stromauf preßbar ist.

8. Injektor nach Anspruch 7, **dadurch gekennzeichnet, daß** das Stellglied (17) durch eine einseitig befestigt W-förmige Feder (18) gebildet ist, deren am freien End angeordneter Schenkel (19) aus einer geneigt zum Pumpenschlauch (7) orientierten Ruhelage in eine parallel zum Pumpenschlauch (7) verlaufende Lage verstellbar und gegen den Pumpenschlauch (7) preßbar ist.

9. Injektor nach Anspruch 8, **dadurch gekennzeichnet, daß** auf der dem Stellglied (17) gegenüberliegenden Seite des Pumpenschlauches (7) als Gegenlager eine Platte (23) angeordnet ist.

10. Injektor nach einem der Ansprüche 7 bis 9, **dadurch gekennzeichnet, daß** ein zweiarmiger Hebel (22), dessen einer Arm in der Ruhelage dem Schenkel (19) am freien Ende der Feder (18) anliegt un dessen anderer Arm mit einem Stellmotor gekoppelt ist, zur Verstellung des Stellgliedes (17) vorgesehen ist.

11. Injektor nach einem der Ansprüche 1 bis 11, **dadurch gekennzeichnet, daß** im Schlauchsystem (5) eine Druckkammer (25) integriert ist, die durch mindestens eine in der Schlauchwandung ausgebildete Öffnung (26) mit dem Inneren des Pumpenschlauches (7) verbunden und von der Flüssigkeit befüllbar ist, und daß die Druckkammer (25) ein unter der Wirkung des Flüssigkeitsdruckes verstellbares Bauteil (27) aufweist, das auf einen Drucksensor (28) einwirkt.

12. Injektor nach Anspruch 11, **dadurch gekennzeichnet, daß** zwischen zwei Abschnitten des Pumpenschlauches (7) ein starres, die Öffnung (26) aufweisendes Rohr (29) eingesetzt ist, an dem die Druckkammer (25) befestigt ist.

13. Injektor nach Anspruch 11 oder 12, **dadurch gekennzeichnet, daß** das verstellbare Bauteil (27) durch eine flüssigkeitsdichte, elastische Membran gebildet ist.

14. Injektor nach Anspruch 11 oder 12, **dadurch gekennzeichnet, daß** das verstellbare Bauteil (27) als gegenüber der Druckkammerwandung abdichtender Kolben ausgebildet ist, der unmittelbar auf den Drucksensor (28) einwirkt.

15. Injektor nach einem der Ansprüche 1 bis 14, **dadurch gekennzeichnet, daß** der Pumpenschlauch (7) zweiteilig ausgebildet ist, und daß der das freie Ende bildende Teil des Pumpenschlauches(7) mindestens ein Rückschlagventil aufweist.

## Claims

1. An injector for administering fluids, in particular contrast agents for X-ray and nuclear spin tomography, having a hose system (5) comprising connecting hoses (6) and a pump hose (7), and a plurality of storage receptacles (4) which are connected by way of a respective one of the connecting hoses and a branching portion (8) to the pump hose (7) leading to a cannula, **characterised in that** associated with each connecting hose (6) are a gas bubble detector (15) and a valve (11) and that associated with the pump hose (7) is a pump member (16) which reverses the flow of fluid from the storage receptacle (4) to the cannula.

2. An injector according to claim 1 **characterised in that** a roller pump (9) is associated with the pump hose (7) for actively conveying the fluid from the storage receptacle (4) to the cannula.

3. An injector according to claim 1 or claim 2 **characterised in that** the gas bubble detector (15) is formed by a ultrasonic transmitter and receiver, by the signals of which the associated valve (11) is switchable.

4. An injector according to one of claims 1 to 3 **characterised in that** the valves (11) are formed by hose clamps (10).

5. An injector according to claim 4 **characterised in that** the hose clamps (10) have a displaceable ram (13) which presses the connecting hose (6) against a counterpart support (12) and thereby constricts the cross-section thereof.

6. An injector according to claim 5 **characterised in that** the hose clamp (10) has a switch (14) for detecting the position of the ram (13).

7. An injector according to one of claims 1 to 6 **characterised in that** the pump member (16) has a control member (17) which bears against the pump hose (7) and by which for the pumping operation the pump hose (7) is deflectable and in the interior of the pump hose (7) the fluid which is upstream of the control member (17) can be pressed upstream.

8. An injector according to claim 7 **characterised in that** the control member (17) is formed by a W-shaped spring (18) which is fixed at one end and whose limb (19) disposed at the free end is displaceable from a rest position oriented in inclined relationship with the pump hose (7) into a position extending In parallel relationship with the pump hose (7) and can be pressed against the pump hose (7).

9. An injector according to claim 8 **characterised In that** a plate (23) is arranged as the counterpart support on the side of the pump hose (7), which is in opposite relationship to the control member (17).

10. An injector according to one of claims 7 to 9 **characterised in that** there is provided a two-armed lever (22) of which one arm in the rest position bears against the limb (19) at the free end of the spring (18) and of which the other arm is coupled to a control motor for displacement of the control member (17).

11. An injector according to one of claims 1 to 11 **characterised in that** integrated in the hose system (5) is a pressure chamber (25) which is connected to the interior of the pump hose (7) through at least one opening (26) in the hose wall and which can be filled by the fluid and that the pressure chamber (25) has a component (27) which is displaceable under the effect of the fluid pressure and which acts on a pressure sensor (28).

12. An injector according to claim 11 **characterised in that** a rigid tube (29) which has the opening (26) and to which the pressure chamber (25) is fixed is inserted between two portions of the pump hose (7).

13. An injector according to claim 11 or claim 12 **characterised in that** the displaceable component (27) is formed by a fluid-tight elastic diaphragm.

14. An injector according to claim 11 or claim 12 **characterised in that** the displaceable component (27) is in the form of a piston which is in sealing relationship with the pressure chamber wall and which acts directly on the pressure sensor (28).

15. An injector according to one of claims 1 to 14 **characterised in that** the pump hose (7) is of a two-part nature and that the part of the pump hose (7) forming the free end has at least one check valve.

## Revendications

1. Injecteur pour l'application de liquides, en particulier d'agents de contraste en tomographie aux rayons X ou à résonance magnétique nucléaire, avec un système de tuyaux (5) formé de tuyaux souples de liaison (6) et un tuyau souple de pompe (7) et avec une pluralité de récipients de stockage (4) qui sont reliés chacun par un tuyau souple de liaison (6) et une pièce de jonction (8) au tuyau souple de pompe (7) menant à une canule, **caractérisé par le fait qu'**à chaque tuyau souple de liaison (6) sont associés un détecteur de bulle de gaz (15) et une soupape (11) et **par le fait qu'**un organe de pompe (16), qui inverse le flux de liquide du récipient de stockage (4) vers la canule, est associé à la conduite souple de pompe (7).

2. Injecteur selon la revendication 1, **caractérisé par le fait qu'**une pompe péristaltique (9) est associée au tuyau souple de pompe (7) pour le transport actif du liquide du récipient de stockage (4) vers la canule.

3. Injecteur selon la revendication 1 ou 2, **caractérisé par le fait que** le détecteur de bulles de gaz (15) est formé d'un émetteur et d'un récepteur à ultrasons par les signaux duquel la soupape (11) associée peut être commandée.

4. Injecteur selon une des revendications 1 à 3, **caractérisé par le fait que** les soupapes sont des dispositifs de serrage de tuyaux souples (10).

5. Injecteur selon la revendication 4, **caractérisé par le fait que** les dispositifs de serrage de tuyaux souples (10) comportent un piston (13) déplaçable qui presse le tuyau souple de liaison (6) contre un contre-appui (12) et diminue ainsi sa section.

6. Injecteur selon la revendication 5, **caractérisé par le fait que** le dispositif de serrage de tuyau souple comporte un contacteur (14) qui détermine la position du piston (12).

7. Injecteur selon une des revendications 1 à 6, **caractérisé par le fait que** l'organe de pompe (16) présente un organe de contrôle (17) appliqué contre le tuyau souple de pompe (7), à l'aide duquel le tuyau souple de pompe (7) peut être dévié pour l'opération de pompage et le liquide situé en amont de l'organe de contrôle (17) à l'intérieur du tuyau souple de pompe (7) peut être refoulé vers l'amont.

8. Injecteur selon la revendication 7, **caractérisé par le fait que** l'organe de contrôle (17) est formé d'un ressort (18) en forme de W fixé à une extrémité, dont la branche (19) côté extrémité libre peut être amenée d'une position de repos en biais par rapport au tuyau souple de pompe (7) dans une position parallèle au tuyau souple de pompe (7) et pressée contre le tuyau souple de pompe (7).

9. Injecteur selon la revendication 8, **caractérisé par le fait que** sur le côté du tuyau souple de pompe (7) opposé à l'organe de contrôle (17) est disposée une plaque (23) formant contre-appui.

10. Injecteur selon une des revendications 7 à 9, **caractérisé par le fait qu'**il est prévu un levier (22) à deux bras, dont un bras, dans la position de repos, est en appliqué contre l'extrémité libre du ressort (18) et dont l'autre bras est couplé à un moteur de réglage pour déplacer l'organe de contrôle (17)

11. Injecteur selon une des revendications 1 à 10, **caractérisé par le fait qu'**une chambre de pression (25) est intégrée dans le système de tuyaux souples (5), laquelle chambre de pression, par au moins une ouverture (26) aménagée dans la paroi du tuyau souple, peut être mise en communication avec l'intérieur du tuyau souple de pompe (7) et remplie de liquide, et **par le fait que** la chambre de pression (25) comporte un élément (27) qui peut se déplacer sous l'action de la pression de liquide et agit sur un capteur de pression (28).

12. Injecteur selon la revendication 11, **caractérisé par le fait qu'**entre deux tronçons de tuyau souple de pompe (7) est monté un tube (29) rigide qui porte l'ouverture (26) et auquel la chambre de pression (25) est fixée.

13. Injecteur selon la revendication 11 ou 12, **caractérisé par le fait que** l'élément (27) déplaçable est formé d'une membrane élastique, étanche au liquide.

14. Injecteur selon la revendication 11 ou 12, **caractérisé par le fait que** l'élément (27) déplaçable est conformé en piston monté étanche vis-à-vis de la paroi de chambre de pression, qui agit directement sur le capteur de pression (28).

15. Injecteur selon la revendication 1 à 14, **caractérisé par le fait que** le tuyau souple de pompe (7) est réalisé en deux parties et par la fait que la partie du tuyau souple de pompe (7) qui constitue l'extrémité libre comporte au moins une soupape anti-retour.
